Europäisches Patentamt

European Patent Office

Office européen des brevets

(11) Publication number: **0 164 836**

A1

## (12) EUROPEAN PATENT APPLICATION

(21) Application number: 85302403.2

(22) Date of filing: 04.04.85

(51) Int. Cl.⁴: **A 61 N 1/00**
A 61 N 5/00, A 61 H 39/00

(30) Priority: 06.04.84 US 597534

(43) Date of publication of application:
18.12.85 Bulletin 85/51

(84) Designated Contracting States:
AT BE CH DE FR GB IT LI LU NL SE

(71) Applicant: Badgley, Laurence E.
370 San Bruno Avenue
San Bruno California 94066(US)

(72) Inventor: Badgley, Laurence E.
370 San Bruno Avenue
San Bruno California 94066(US)

(74) Representative: Collier, Jeremy Austin Grey et al,
J.A.Kemp & Co. 14, South Square Gray's Inn
London WC1R 5EU(GB)

(54) Combination of vascular autonomic signal treatment and analyzing means.

(57) A combination for use in treating an individual includes means for establishing a location of an energy field vortex (12) on the individual. The energy field vortex is analyzed to determine at least one characteristic of the vortex. The combination also includes means for introducing into the energy field vortex a stimulus, such as light, sound, a magnetic field, an electrical signal, or the like, which produces a positive change in the individual's vascular autonomic signal. The stimulus is removed when at least one characteristic of the energy field vortex achieves a desired state.

FIG._2.

## VASCULAR AUTONOMIC SIGNAL TREATMENT METHOD

### BACKGROUND OF THE INVENTION

1. **Field of the Invention.** This invention relates to a novel treatment modality in which a nervous system response known as the Vascular Autonomic Signal (VAS) is used in a feedback loop during the treatment. More particulary, it relates to such a treatment modality in which VAS response is used to bring energy fields generated by the body into balance.

2. **Description of the Prior Art.** Throughout history and in many different cultures records have been made of the observation of aura fields and chakra fields about the human body, as described in Leadbeater, C.W., **The Chakras**, The Theosophical Publishing House, Wheaton, Illinois, 1927. It is probable that the different names for the energy field, shown in the following table, each identify the same energy field or various qualities of the same energy field.

| | |
|---|---|
| Aura: | Metaphysicians of Western European tradition; W. Kilmer |
| Bioplasma: | Contemporary Russian |
| Chi: | Chinese |
| Life Force: | Vitalists of pre-1900's European and American scientific tradition |
| Orinda: | American Indian |
| Odic Force: | C. von Reichenbach of Germany |
| Orgone: | Wilhelm Reich of Germany |
| Prana: | Indian (Hindu) |
| Chakra: | Indian (Hindu) |

These observations were often made by persons with clairvoyant abilities. Scientific methods to visualize these fields are disclosed by Kilner, Walter J., **The Human Atmosphere, the Aura Made Visible by the Aid of Chemical Screens**, Rebman Company, New York, 1911. The field was measured 25 feet out from the human skin surface by Bose, Sir Jagadis Chandra, **Collected Physical Works**, Longmans, Green and Company, Ltd., London, 1927.

The chakras have been described by clairvoyants as whirling disks of diaphanous colors which seem to emanate from discrete anatomical locations along the anterior midline of the human body. Seven major chakras have been described, named and given a number. Each chakra has been related to a predominant color and the same numbered chakra in different individuals usually has the same coloration. Based on anatomical correlations, each chakra has been identified with a gland, and the energetic nature (color, sound vibratory rate, etc.) of each chakra is thought to relate to the physiological status and functioning of the gland to which it corresponds.

The state of the art in the analysis and use of such energy fields in treatment modalities is described by Norgier, Paul, M.D., **From Auriculotherapy to Auriculomedicine**, Maisonneuve, France, 1983. While a substantial body of literature and investigative work has been created and carried out in the past, much remains to be done in order to understand the nature and uses of these fields in treatment of abnormal conditions.

**SUMMARY OF THE INVENTION**

Accordingly, it is an object of this invention to provide an improved treatment method which relies on the analysis and modification of energy fields emanated from the body to bring them back into balance.

It is another object of the invention to provide such a treatment method in which the vascular autonomic signal is employed in a feedback loop to monitor the effectiveness of the treatment.

It is a further object of the invention to provide such a treatment method for preventing imbalances in energy fields emanaging from the human body.

The attainment of these and related objects may be achieved through use of the novel treatment method herein disclosed. A treatment method for an individual in accordance with this invention includes establishing a location of an energy field vortex on the invidual. The energy field vortex is analyzed to determine at least one characteristic of the vortex. A stimulus which produces a positive change in the individual's vascular autonomic signal (VAS) is introduced into the energy field vortex. The stimulus is removed when the at least one characteristic of the energy field vortex achieves a desired state.

The general body aura energy field and the discrete chakra fields have electromagnetic qualities. Many disease states can be related to abnormal chakra characteristics. Further, many disease states can be beneficially influenced by the balancing of disordered chakras. Balancing of chakras can be obtained by measured applications of resonant frequencies of light, sound, magnetism, crystal transduced human aura, flower essences, herbs, food, cell salts, homeopathic remedies and other natural energies. During or following such applications, the chakra fields are again analyzed and the application terminated when a desired state of the chakra fields is observed.

The attainment of the foregoing and related objects, advantages and features of the invention should be apparent to those skilled in the art after review of the following more detailed description of the invention, taken together with the drawings, in which:

## BRIEF DESCRIPTION OF THE DRAWINGS

Figure 1 is a frontal view of a human body, useful for understanding the invention.

Figure 2 is a side view of a portion of the human body shown in Figure 1, useful for a further understanding of the invention.

Figure 3 is a schematic representation of a human body oriented in different positions during use of the invention.

Figure 4 is a perspective view of an energy field vortex emanating from a human body during use of the invention.

Figure 5 is a perspective view of a human body during use of the invention.

Figure 6 is a perspective view of a human body in different orientations during use of the invention.

## DETAILED DESCRIPTION OF THE INVENTION

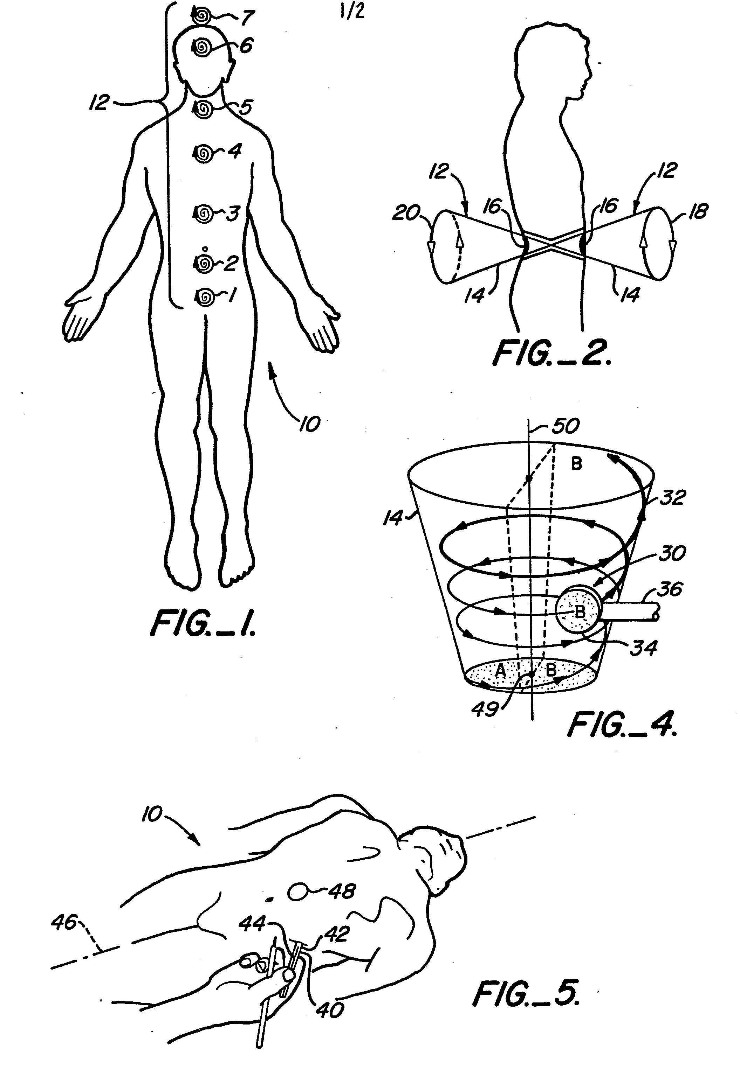

Turning now to the drawings, more particularly to Figure 1, there is shown a human body 10, with seven chakras 12 shown as spirals at their locations on the body 10, as located in the substantial literature of the prior art describing them. The chakras 12 are traditionally numbered from 1 to 7 in ascending order as shown. The seven chakras 12 have the characteristics shown in the table below.

| Number | Name | Color | Sound | Gland |
|---|---|---|---|---|
| 7 | crown | purple | G | pineal |
| 6 | brow | indigo | F | pituitary |
| 5 | throat | blue | E | thyroid |
| 4 | heart | green | D | thymus |
| 3 | solar plexus | yellow | C | pancreas |
| 2 | spleen | orange or pink | B | adrenal |
| 1 | root | red | A | gonads |

Table 1. Characteristics of the chakras.

1. Reading the Vascular Autonomic Signal (VAS)

In order to test the functioning of the chakras, the practitioner needs skill at reading the VAS. The following discussion covers this subject.

The living organism is an infinitely complex mass. Trillions of cells are spacially related and interdependent in their function. Each cell contains millions of molecules involved in thousands of known chemical reactions. The whole arrangement functions smoothly even though a significant part of its mass experiences disintegration of its structural form each day and has to be replaced. For example, the entire red blood cell population is gradually replaced over a few months time and the cellular lining of the gastro-intestinal tract is replaced every few days. The dynamic balance necessary for the success of such a complex system strongly infers the guiding influence of a field of energy which pervades the entire mass of the organism.

Throughout history certain scientists have approached their study of the organism with the proposition that an all-pervasive energy controls organism functioning. Their method of study has often been to measure change in organism function when change occurs in a measurable quality of the natural ambient field. Abrams demonstrated beyond doubt the subtle changes in fields of electrical energy that correlate with human tissue disorder in Abrams, Albert, M.D., **New Concepts in Diagnosis and Treatment**, Physico-clinical Company, San Francisco, 1922. Harold Saxton Burr related physiological functioning to changes in the ambient electrical field in Burr, Harold Saxton, Ph.D., **Blueprint for Immortality, the Electric Patterns of Life**, Neville Spearman, London, 1972. George Starr White proved the relationship of organism function to radiant colored light in White, George Starr, M.D., **A Lecture Course to Physicians on Natural Methods in Diagnosis and Treatment**, Los Angeles, 1918. More recently Russian scientists have established a large body of scientific evidence for the relationship of geomagnetic ambient fields to human and subhuman behavior and health in Dubrov, A. P., **Geomagnetic Field and Life**, Plenum Press, New York, 1978.

In the medical sciences the German electro-acupuncturists have gathered scientific evidence for the influence of natural energies of homeopathic substances on human functioning as reported by Leonhardt, Horst, M.D., **Fundamentals of Electroacupuncture According to Voll**, Medizinisch Literarische Verlagsgesellshaft MGH, Uelzen, 1980. In the last twenty years, Dr. Paul Norgier of France has exquisitely demonstrated the ability of subtle energy fields to influence living tissue in Norgier, Paul, M.D., **From Auriculotherapy to Auriculomedicine**, Maisonneuve, France, 1983.

A common characteristic of the measurements by the scientists mentioned above is that these measurements were made on living organisms. On many occasions the parameters of change measured in the living organisms were tissue or organ reflexes. When one desires to measure organism change induced by a field change a useful parameter to monitor is a body reflex. Reflexes of the muscular system are especially useful since they are rapid in onset and rapid in diminishment. Smooth muscle is especially suitable for the monitoring of muscle reflexes. The smooth muscle tonicity of visceral (intestinal) tissue can be monitored by the percussion techniques fostered by Abrams and George Starr White. Smooth muscle of vascular (arterial) tissue can be monitored by palpation as popularized by George Starr White and Paul Norgier or by instrumentation such as used by George Starr White.

Smooth muscle is innervated and controlled in its function by the autonomic nervous system. Medical science identifies the autonomic nervous system as that part of the nervous system which keeps tissue and organs functioning in balance with and adapted to the environment. The autonomic nervous system originates in the hypothalamus section of the brain and a large part of its function is independent but not isolated from the brain centers of voluntary control. The hypothalamus is the origin of two branches of the autonomic nervous system--the parasympathetic and the sympathetic. The parasympathetic stimulates reflexes which are associated with relaxation, digestion, sexual function and which are generally conserving of body energy. The sympathetic nervous system stimulates physiological functions which cause the body to adapt to new and threatening environmental circumstances. Sympathetic nervous system function evokes dilation of peripheral blood vessels so that blood can be more plentiful in the extremities, which need instant energy (sugar and adrenalin) as the organism fights or flees. Dr. George Starr White has called this rapid dilation of peripheral blood vessels the "vascular response." Dr. Paul Norgier has named the increased tonus of the arterial wall the "vascular autonomic signal" or "VAS". The word "VAS" is used in this application to denote the palpable increased tension in pulse pressure which occurs when the body field is approached with an energetic stimulus.

The VAS has certain important characteristics:
(1)   onset is instantaneous;
(2)   change is easily monitored;
(3)   it occurs in a living organism;
(4)   it gives information about the stimulus;
(5)   it gives information about the organism.

When a positive VAS occurs it means that the organism can successfully adapt to the stimulus which is initiating it. A simplistic interpretation of a positive VAS might be that the organism likes the stimulus that it is experiencing and will be benefited by it.

Once a practitioner has learned to read the VAS he can make decisions about the size and quality of body field disorders in clients. Subtle energy stimulations such as light, magnetism, electricity and sound all influence the degree of contractability (tension) of smooth muscle. VAS interpretation provides information about the quality and quantity of energy needed to alter a disturbed body field.

A convenient way to measure the VAS is to palpate the radial pulse of a client who is lying with his head in the earth west or east direction. The pulse can be palpated with the examiner's thumb or other fingers. A good practice position to start with is for the practitioner to place the end of his thumb touch pad on the radial artery. The practitioner's thumb should be slightly less than a 90 degree angle with the client's arm and should not be angled off to either side. The practitioner should first press with enough force to find the pulse, and then he should relax that force until he barely feels the pulse pressure. At this point the examiner might visualize that his palpating finger is only slightly indenting the arterial wall which is closest to his examining finger. Now the practitioner is ready to appreciate the change in arterial wall tension when the client's body is presented with a test stimulus.

There is a convenient way for the beginner to observe the VAS change while he is practicing alone. The earth's magnetic field is directed from south to north. The magnetic flux passes from south to north at a strength of 0.5 gauss. The magnetic flux penetrates all matter with few exceptions. As the flux passes through the human body it causes stimulation of the autonomic nervous system. The degree of stimulation is a function of the orientation of the nervous system in the earth field. A human facing north or south is stimulated to a greater degree by the earth's magnetic field because more of the nervous system antenna is exposed to the south to north magnetic flux. The beginner can undertake the following exercise in order to study the VAS:

(1) Stand facing east or west;

(2) Take your own pulse as directed above.

(3) Turn body to north-south meridian without moving hand positions;

(4) At the moment of attaining the north-south position the pulse tension will increase (positive VAS).

Various phrases might describe the pulse change that is called the positive VAS:

(1) more full;

(2) greater tension;

(3) strikes the palpation finger with more force;

(4) each pulsation seems firmer, harder or more peaked;

(5) slightly banging;

(6) bounding.

As the beginner turns from the north-south meridian to the east-west direction, the negative VAS will be palpated. The negative VAS might be described as:

(1) more damped;

(2) strikes palpation finger with less tension or force;

(3) backs off or disappears;

(4) each pulse peak is less sharp;

(5) non-bounding;

(6) softer.

With practice the student will gain confidence in the reproducibility and reliability of the VAS. Please note that the VAS pulse change is **not** a rate or rhythm change.

Another characteristic of the positive VAS is that the palpable pulse change often lasts for approximately five to twelve beats and then fades. This is compared to the negative VAS which sometimes has one or two strong beats after the onset of the test stimulus and then suddenly drops to the negative VAS mode. The difference between the positive and negative VAS is easily recognized in any single test situation by simply turning the test stimulus into and out of the aura field being tested and the practitioner looks for relative change in the pulse beat character.

In this application VAS means the positive VAS. The VAS indicates that the body can adapt to the stimulus. It is the practitioner's goal to identify loci or regions in need of energy stimulation and to identify beneficial poses and frequencies of stimulation. Therefore on most occasions the practitioner will work to the positive VAS or simply VAS.

A good stimulus to begin learning with is a three-phase filter. The three-phase filter can be brought toward the client's ear. When the filter reaches a discrete distance from the ear, more prominent pulse beats will be immediately palpable. The distance from the ear at which the filter evokes a positive VAS is measured in inches and will be constant distance from the ear during a single session. It must be kept in mind that the change being observed is a change in tension of the pulse beat and not a change in pulse rate. The three phase filter was chosen for beginning practice because all persons experience a positive VAS when this filter enters the aura around their ear, as taught by Norgier.

Another stimulus response is constant in its effect in most right-handed people and is a useful training method in beginning practice. A green light shined on the right ear or a red light shined on the left ear of a client evokes a positive VAS. Just as with the three-phase filter, if the light is suddenly aimed away the VAS will immediately drop out or fade after one or more firm beats. The three-phase filter effect demonstrates the body sensitivity to the intersection of its emitted light (aura) by an artificial color. The radiant color effect demonstrates the body sensitivity to direct stimulation by different frequencies of radiant colored light.

The practitioner should keep in mind that the VAS is a relative measure. It is identified by the change in pulse tension that occurs at the onset of stimulation. It is not possible to identify the positive or negative VAS as absolute or isolated observations because the variability in tonus is too subtle.

Encruitment

Understanding of the phenomenon of encruitment is crucial to making precise readings of the VAS. When a new stimulus enters the aura field, the autonomic reflexes respond immediately but sometimes with a short period of oscillation. The first exposure of a single stimulus might cause a positive VAS and then upon immediate removal and reintroduction might cause a positive VAS and then upon immediate removal and reintroduction, the stimulus might cause a negative VAS. After one more removal and reintroduction, the VAS will be positive again and remain positive to all subsequent reintroductions of the same stimulus into the field during the test session. The initial variability of response to a stimulus is termed an oscillation. The phenomenon described suggests that the body needs to experience both the presence and a secondary absence of a stimulus in order to differentiate the two energy poles of a stimulus. An analogy might be the need to program a computer with informtaion before the computer can be used to make decisions.

The meaning of the phenomenon of encruitment to the VAS observer is that he must be patient and not rely on the very first pulse beat or beats for the definitive VAS information he seeks. Rather, he should repeat the test stimulus a few times so as to reinforce the response. This delay might introduct 3 or 4 extra seconds into the taking of each reading if such oscillations appear. Oscillations present no great problem to the VAS observer if he is aware of them. Once the stimulus-response circuit has been reinforced, the VAS response is constant and dependable, and the practitioner will not fail to have confidence in it. One exception to this statement might be that a female who is menstruating has energy oscillations which might interfere with reliable data collection.

Occasionally a client will present a subdued radial pulse which is difficult to feel. The pulse can be rendered more prominent by having the client make a fist with his hand. The fist grasp that the client makes should not be strenuous and does not even need to be firm.

Another technique to make the pulse more palpable is for the practitioner to take the pulse with his middle and index fingers as he grasps the client's wrist with his entire hand curled around the client's wrist. In this method it seems that the practioner's curled, grasping hand becomes a more broad area of sensitivity to pick up the client's pulsation changes than just the smaller surface area of the practitioner's thumb digit pad.

The following discussion shows how to use the VAS to identify body field disturbances and to identify the energy pole and frequency which balances field disorders.

2. The Chakra Energy Field.

As shown in Figures 1 and 2, chakra fields 12 are discrete vortexes 14 of energy within the total body aura field. The chakra fields 12 are electromagnetic regions in which the energy flows in a spiral outward from the body as opposed to other body regions where the outward flux is generally linear. The chakra field projection 16 on the skin surface is disk shaped and usually one to three inches in diameter. From the skin surface the field extends as an upside down truncated cone 14 with a contour slope which is constant in one chakra 12 but varies approximately between 45 and 70 degrees in different chakras.

The electromagnetic flux in a chakra field 12 has an outwardly directed spiral path. The direction of the spin is either clockwise as shown at 18 or counter-clockwise, as shown at 20. In the normal female with head west, the anterior body chakras are usually clockwise. The opposite spin is often found in the normal male.

Chakra vortexes 14 exist in pairs with the anterior vortex usually spinning in a direction opposite to the posterior vortex as both vortexes are viewed by a stationary distant observer. This chakra pair is termed "flipped" in their spin directions.

A disordered chakra will often be seen to have both the anterior and the posterior vortexes 14 spinning in the same direction as both vortexes are viewed by a stationary distant observer. This chakra pair is said to be spinning "in concert."

As the chakra vortex leaves the body it passes through the skin envelope. Using the tools and methods described in this application, it is possible to observe and diagram the chakra's disk-shaped cross-sectional area 16 where it cuts through the skin envelope. This disk area 16 is called the "vortex disk."

At the skin surface, the vortex disk 16 of the anterior chakra is usually of lesser diameter than the vortex disk 16 of the posterior chakra. This observation might reflect the more proximal location of the gland of origin of the chakra vortex to the anterior body surface and that the vortex borders have had greater divergence by the time they cross the posterior skin envelope.

The practioner should become familiar with the different spin directions of the chakras when the client is lying down face up and with his head in different compass directions. The spin directions of the normal femal are given in Figure 3.

It is notable that when the head is directed north, as shown at 22, the chakras 12 do not have a measurable spin direction. The life energy is quiescent and possibly the body is functioning in an energy conserving mode. In the head-north direction, the longitudinal body magnetic field is aligned in series with the earth's magnetic field. Perhaps the body is absorbing energy and is being recharged in this orientation. These observations provide evidence for the usefulness of sleeping with the head pointed north.

Figure 3 shows the normal anterior body chakra spin as "toward the head and then north" in both the head-east and head-west orientations, shown at 24 and 26, respectively. The implications of these observations for an understanding of the direction of flow of body energy are enormous. In summary, since spin direction in both the head-east and head-west orientations 24 and 26 is constant relative to the earth's magnetic field, then it can be concluded that the life energy of the body has two characteristics:

(1)  The life energy is influenced by the earth's magnetic field;

(2)  The life energy flows longitudinally between the head and the feet.

After he becomes famiiar with the dynamics recorded in Figure 3, the practitioner will understand the necessity of measuring and balancing the client while the client's head is in a earth-east or earth-west direction 24 or 26. In practice, the head-west direction is usually chosen.

The measurements made with the subject's head placed south, as shown at 28 indicate that the chakra spins are randomly alternating. This indicates that the intersection of the body longitudinal flux and the earth field flux is dissonant, non-resonant, and without a dominant resultant direction of spin. The head-south position 28 is disorganizing to the body field and one should never sleep in this direction.

In keeping with the universal law of magnetism that unlikes attract, it is hypothesized from the data presented above that the head is the south pole of the body magnetic field and that the feet are the north pole of this field.

The following discussion introduces the tools which can be used to identify the chakra characteristics outlined above.

### 3. Measurement Tools.

**Natural Quartz Crystal**

The quartz crystal is discussed first since it is the most versatile instrument. In its natural form the quartz crystal has six sides which join to a peak consisting of six facets. The quartz crystal is a one-way transducer of aura energy. It picks up the aura of the hand that holds it or other aura nearest its base and directs it out the peaked end in a straight line energy beam. This focused beam is called the "pointed crystal beam" and is along an axis which is continuous with the internal longitudinal axis of the crystal. The energy of this beam is probably not polarized. Aura energy passing through a crystal held on its side exits the side away from the hand that holds the crystal. This side beam is not focused like the pointed crystal beam and the energy of the side held crystal beam is polarized along an axis which is parallel to the long axis of the crystal. This side beam is called the "polarized side beam."

The pointed crystal beam evokes a VAS response when it intersects a region of aura energy intensity such as a vortex. A crystal beam pointed tangentially into the vortex evokes the VAS when the beam strikes the spiral flux in such a fashion that the crystal beam hits the spin flux as it rotates away from the crystal. When the pointed beam strikes the spiral flux head-on as the spin turns toward the pointed crystal beam a negative VAS is aroused.

A crystal beam pointed at a region of chi concentration such as an acupuncture point or an energized acupuncture meridian evokes a VAS response. It is believed that this phenomenon was the basis for the discovery of the acupuncture meridian system by the ancients. A crystal beam charged to its full strength by the techniques described below passes completely through the body it is aimed at and this beam can be used to trace out areas of concentrated energy internally. The pointed crystal beam can be used to locate the internal focus which gives rise to an efflux energy, which is usually organized into a vortex.

It is important that each practitioner learn for himself the reality of a quartz crystal beam, and the exercises listed here are a good starting point. The reality experienced during these exercises will dramatically heighten the practitioner's awareness of the body's subtle energy field. The exercises described were developed by Marcel Vogel, a foremost authority on crystal healing energies.

The practitioner should obtain a quartz crystal which has surfaces and corners which are relatively undamaged. The crystal should fit comfortably in the curl of the hand and fingers and the one-half of the crystal nearest its tip should be relatively clear and unflawed by opaque growth lines and fractures.

Usually the practitioner holds the crystal in the right (the sending) hand. To prepare the beam the fingers of the right had are used to caress the crystal and to rotate it in the right hand with the fingers and thumb of the right hand. After a few turns the practitioner will note that the surface texture of the crystal has become slightly sticky. At this point the rolling motion can be stopped and the crystal held in the hand's curled grip.

Now point the crystal tip at the left hand which is held outstretched and approximately four to six inches away from the right hand which holds the crystal. Begin to rotate the entire right hand with its firmly grasped crystal in a clockwise fashion so that the imagined pencil-thick beam from the crystal tip plays upon the screen provided by the left palm and fingers. The practitioner should be comfortable, relaxed and contemplative. He should continue the right hand rotations and concentrate on the detection of subtle sensations in the left hand. These sensations will occur at the point of intersection of the crystal beam on the hand and will have different characteristics in different people. Variously these sensations have been described as: coolness, warmness, slight wind, tingling, crawling, shifting pressure, and similar to electricity.

With practice the practitioner will learn the distinct feeling of the crystal beam. After this has been accomplished the practitioner should allow a second person to put his outstretched hand between the practitioner's two hands so that the second person can feel the beam. The practitioner will note that the beam passes through the second person's hand without diminution in the magnitude of its stimulation strength. Now the practitioner can back away from the second person's hand while keeping the crystal hand rotating and pointed at the second person's hand. The second person will be able to confirm the effective passage and reception of the crystal beam over many feet of airspace. Finally, the practitioner can demonstrate that the crystal beam passes through the human body and through non-living objects such as books and tables without diminishment in the magnitude of its sensibility.

Section 7 below describes the method of using the quartz crystal to heal body energies.

**Magnets**

Magnets function as life energy pumps. The south pole attracts life energy and speeds it up. The north pole repels life energy and slows it down. When the vibrations of light are faster, then the color of the light does to the green end of the spectrum. When light is slowed down in its vibratory rate it does toward the red end of the spectrum. By convention the south pole of a magnet is often labeled red and the north pole green.

The field of force coming out one end of a magnet turns back on itself to enter the other end of the magnet. If it could be visualized this field would have the shape of a fountain of water. When a polarizing filter is placed on the end of a magnet the magnet field is given a laser-beam-like quality, and the beam shines along an axis which is continuous with the longitudinal axis of the magnet. A magnet fitted with a polarizing filter is called a reticulated magnet and was proposed by Dr. Paul Norgier. The beam of a reticulated magnet resonates with the body energy when it is beamed at the body chakra fields 14 and the VAS is aroused. This arousal is instantaneous as the beam enters the chakra vortex field 14 either from above or as the beam hits the vortex 14 spin head on. The reticulated magnet beam can be used to locate the invisible (to other than clairvoyants) borders of chakra energy fields. The end with the filter fixed on it is the end which should be aimed at body energy fields.

A magnet wand 30 (Figure 4) is a convenient tool with which to determine the direction of the chakra vortex 14 vortex spin 32. Life energy is drawn to the south pole of a magnet and is repelled by the north pole. The magnet wand 30 is a small disk magnet 34 with a non-magnetized wood handle 36. The magnet wand can be introduced into the chakra vortex field 14 and thereby influence the vortex spin 32 and the rate of energy efflux from the body. Vortex spin 32 is enhanced by an orientation of the magnet where the spin goes into the south pole and out the north pole, and the VAS is aroused by this magnet orientation.

The magnet wand can also be used to determine the exact point center fo a chakra skin disk, and the method of doing this is described below.

Before the practitioner uses any magnet, he should first test the magnet for the correctness of labeling of its poles. This is easily done using a compass. Keep in mind that every compass needle is a tiny magnet. Remember that with electric and magnetic energy likes repel and unlikes attract. This is a fundamental law of nature and must be kept in mind throughout all energy work. The south pole magnet end of the compass needle is actually the needle end that points north. Unlikes attract and the needle south pole is attracted to the earth north pole. The north pole magnet end of the compass needle is actually the needle end that points south.

In order to test any magnet for the correct pole simply direct the pole in question at the compass. The north seeking end of the compass needle will be drawn to the north pole of the magnet you are testing. The south seeking end of the compass needle will be drawn to the south pole of the magnet you are testing.

### 4. Making the Measurements.

In Section 2 above some of the characteristics of the normal chakra field were discussed. This section covers some methods of measurement of the functioning of the chakras.

The client should be unclothed and lying on his back with his face up and his head west. All jewelry and gemstones should be removed. All magnets should be at least ten feet away from the client and not pointed at the client. Magnets should be brought into the client's aura field one at a time as they are needed for making measurements. The essences should be a few feet away from the client and within easy reach of the practitioner. All color filters should be lying on their side parallel to the floor excepting when they are used one at a time in the client's chakra field. The inventor routinely positions the client with head west and the measurements in this description are given according to this reference except where indicated. The practitioner should position himself so that he is near the client and able to grasp the client's wrist with one hand and scan the client's anterior body with his other hand, which will hold a measuring tool. During all measurements it is very important that the practitioner be relaxed and not experiencing any muscle straining due to an odd position of his arm or hand which is taking the client's pulse. Any muscle strain or discomfort in this arm will render the VAS reading exceedingly difficult.

The first tool to use is the side-held crystal, and this should be slowly scanned along the anterior longitudinal midline of the client, about three to six inches above the client's skin surface. The crystal longitudinal axis should be perpendicular to the client's longitudinal axis.

At discrete locations along the patient's longitudinal midline axis the VAS will be stronger. This location is called a "crystal node" and it represents a chakra's location. As the crystal passes over this location the VAS intensity will build, peak, and taper analogously to the sound intensity on a radio station as one dials toward, at and away from the exact point on the dial where the station comes in the loudest. The practitioner should mark the approximate positions of the most intense VAS locations onto the skin with a felt pen.

After the first six chakras (the seventh chakra measurement is described separately below) have been approximately located, the reticulated magnet 40 (Figure 5) can be positioned with its front tip 42 two to four inches above the client's skin surface and held so that it is aimed at the client's body 10 with its longitudinal axis 44 perpendicular from above to the longitudinal axis 46 of the client's body. Next the reticulated magnet beam is used to scan the region around the pen mark which already marked the crystal node. With careful scanning a region 48 can be delineated which is approximately one to three inches in diameter and which is disk-shaped. Within the borders of this region the VAS will occur. This disk represents the projection of the chakra vortex on the surface of the client's skin as the chakra field passes through the skin envelope. Pen marks can be put on the skin to delineate the approximate borders of this disk. The pointed crystal beam can be used to perform the same function as the reticulated magnet function in finding the vortex disk.

The final skin marking to make is the exact center point of the chakra disk. The magnet wand 30 is used to find this locus. The wand is slowly passed along an imaginary line which is longitudinal through the vortex disk. This disk diameter is called the "longitudinal disk diameter." Along one-half of this disk diameter the VAS will occur. At the exact point where the VAS drops off a mark should be made. Next, the same reading should be made along another imaginary disk diameter which is perpendicular to the longitudinal disk diameter. This disk diameter is called the "transverse disk diameter." Again an exact point will be located where the VAS changes, and this point should be marked. It will be found that the markings on the longitudinal and transverse diameters will overlap at a single point which represents the exact center point 49 (Figure 4) of the chakra disk. This point lies on the chakra's imaginary vortex axis 50 as it passes superiorly out of the client's body.

While making the measurements along the longitudinal and transverse diameters mentioned above the practitioner should note which is the north pole side of the magnet wand. The chakra vortex spin direction can be determined from this observation because the VAS occurs when the magnet wand magnet is aligned within the vortex so that the vortex spin enters the south pole and exits the north pole (Figure 4). The vortex spin is in the direction from the south to the north pole of the magnet wand. As the spin direction is noted it is conveniently recorded by placing an arrow head mark on the vortex disk 48 circumference, which was already marked on the skin.

By now the practitioner has delineated the physical borders of the chakra energy field, the exact center of the chakra disk on the skin surface and the direction of the chakra spin. It is an easy task to introduce colored filter gels, essences, sounds, herbs, salts, etc., into the chakra field to see which frequencies of these substances the body responds at each chakra. In the normal healthy individual the frequencies should be in Table 1. In the female with the head west the anterior body chakra spin is often clockwise, and in the male with head west the spin is often counter-clockwise. The practitioner must keep in mind at all times that the discrete frequencies of energy which give rise to the VAS in a chakra are the frequencies which that chakra needs in order to maintain or regain balance.

The next section describes the methods of providing energies to disordered chakras so as to rebalance them.

### 5. Balancing the Chakras.

Different qualities of energy can be used to balance the chakras. The practitioner's aura field can be used by means of the natural quartz crystal transducer. This method is described in Section 7, "Crystal Healing." The other energies are described in this section.

Color frequencies can be effectively provided by radiant colored light. Colored clothes and colored gem stones can be worn in the chakra field, but the most powerful treatment is to use the exact frequency of radiant colored light. A white light lamp can be fitted with colored filter gels so as to shine the discrete frequency of a balancing color into the chakra energy field. For this purpose the practitioner can employ a metal holder which can be clamped to a tensor type of lamp in order to hold colored filter gels which will transmit light of the desired frequencies.

Electromagnetic energies such as light, electricity and magnetism can each be used to balance chakras. These energies are most effective if their stimulation is pulsed at a regular frequency. It is especially preferred to pulse magnetic energy and light energy at a frequency which is given by the client's own pulse. This frequency has been found to be a very effective universal frequency of stimulation in natural healing techniques.

Magnetic energy can be used to balance chakras which have a deviant spin direction. The south pole magnet is used to create a counter-clockwise spin. Keep in mind that the anterior chakra spin in males is counter-clockwise and the posterior chakra spin is often clockwise. The opposite directions are often found in females. These male and female spin directions are not universal, and individuals need to be examined in order to ascertain their predominant and normal spin directions. Permanent magnets can be taped with the appropriate pole toward the skin and directly over the chakra skin disk center. Oscillating electromagnets seem to be very effective for obtaining chakra spin balance.

Disk magnets can be used to balance chakra fields. The choice of which pole to tape next to the skin at the vortex disk center can be made by using the magnet wand 30. Alternately, the north and south poles of the magnet wand can be presented to the vortex disk center. That pole which gives the VAS is the treatment pole to use. A similar measurement can be made on the posterior chakra of a chakra pair, and two magnets can be taped to the body--one anteriorly and one posteriorly.

**Color Filters**

A set of seven color filters may be employed which has been chosen because of the resonance of these colors with normal chakras. Table 2 shows the colors of the set.

| | |
|---|---|
| 034 | Medium blue |
| 043 | Yellow green |
| 031 | Daylight blue |
| 045 | Medium red |

| 004 | Lemon yellow |
| 029 | Medium purple |
| 011 | Magenta |

**Table 2. Color Filters**

The specific light frequency qualities of each filter have been measured by spectrometric methods. These color filters are commercially available from Edmund Scientific Co., Barrington, New Jersey, and further information on their light transmissive properties is available in publication No. 9081, **Spectral Curves**, available from Edmund.

The resonance qualities of a filter are best observed by placing the filter horizontally in the chakra field and approximately two inches above the body. Below this level the VAS response is not as dramatic. The practitioner must remember that each chakra field is a truncated vortex with its truncated end flush with the skin surface. As the filter gel is kept horizontal to the client's body and passed into and out of the field, the VAS response will arise as soon as the leading edge of the filter gel contacts the contour of the vortex. As the filter is continued through the vortex the VAS response continues until the trailing edge of the filter exits the vortex field. The VAS response at the entrance and exit points of the vortex is abrupt, and this observation dramatically demonstrates the sharp contours of chakra vortex fields. The first time the practitioner makes this observation he will assuredly be amazed.

Human beings are afflicted with many ills. Some particularly devastating diseases are not well understood. The practitioner should use an orange color filter in the measurement of chakra fields in individuals with devastating diseases. The orange color filter can also be used to meausre the aura field around afflicted body regions of individuals with devastating diseases. The appropriate frequency of orange color filter is also commercially available from Edmund Scientific Co.

## Frequencies

It has long been observed by various investigators that when one desires to influence the body's subtle life energy field the external stimulating energy is most effective when it is presented in regular bursts. The scientific way to describe this is that the energy is delivered with a certain frequency. The shorter the time between bursts, the higher the frequency. A unit of measure of frequency is the hertz, one hertz is one burst or cyle per second. Electro-acupuncture stimulation has generally been found to be effective at frequencies between one and ten hertz. An effective frequency with which to stimulate the subtle life energy field is the frequency of the human respiratory rate or the human pulse rate. Respiration cycles at approximately 0.3 hertz and the pulse cycles at approximately 1.3 hertz.

Effective stimulation rates can be provided by hand-held energy tools like the magnet and the natural quartz crystal by pulsing these tools. Pulsing is obtained by rotating the tool in such a fashion that the energy beam of the tool cycles as it is directed at the chakra which is intended to be balanced. The hand is rotated in a circle so that the tool's energy beam traces a circle as it intersects the chakra. One rotation of the hand completed approximately every second is an effective stimulation frequency for balancing.

## Antennas

Over the centuries acupuncturists have used metal needles to balance life energy. The present investigations lead to the conclusion that acupuncture needles are antennas for energy transmission into and out of the body. The acupuncture effect can also be easily obtained by applying metal pellet antennas to the physical body-aura interface, i.e., the skin surface.

Various metals can be used and various metals have different effects on the life energy. Gold and silver have useful energetic qualities. The most utilitarian metal is iron. Iron seems to bring into balance either life energy excess or life energy deficiency. A simple method of antenna application in chakra balancing is to tape a small steel pellet to the chakra disk center for a few days until chakra balance is regained. The flower essences can be applied to the outside of the tape or to the chakra disk around the pellet tape so as not to interfere with the sticking quality of the tape.

Sound

Sound can be used to stimulate chakra balancing. Tuning forks can be vibrated and directed into chakra energy fields. Hand held tuning forks should be directed so that the tuning fork head is horizontal and so that the sound waves enter the field along a north-south axis. This orientation gives the best VAS response. That discrete tuning fork frequency which evokes the VAS response is the treatment frequency. The balancing treatment application can be either provided by the same orientation of the tuning forks or by placing the base of the tuning fork directly onto the vortex disk center. Sound energy frequencies may be supplied in pulses in time with the client's pulse.

Essences, Herbs, and Cell Salts

Essences, herbs and cell salts are easily introduced into the chakra field in order to measure their effect on life energy. A VAS response will indicate the most effective essence or herb. The essences may be chosen because they have been found to resonate with the individual chakras of normal fields. During a measurement session these essences can be delivered to the field by placing the closed vial into the field being measured. Electromagnetic life energies pass through quartz glass so that the vial lid need not be removed. For balancing applications, a small amount of the oil can be placed on the chakra disk center twice daily until the chakra regains balance. Balance of a chakra is evidenced by correct spin direction, and by correct color and essence resonance (see Figure 1 and Table 1).

Throughout this book attention has been given to the measuring and balancing of the chakra vortex that emanates superiorly from the body. The same chakra focus that gives rise to an anterior vortex gives rise to a posterior vortex (Figure 2). The posterior vortex of a disordered chakra holds its spin and other energy characteristics when the client turns over on his stomach. The posterior vortex is directed superiorly in the person lying on his stomach with his face down.

The chakra vortex disk centers of both the posterior vortex and the anterior vortex will be on a line drawn perpendicularly through the body and they will directly overlie each other. The first time the practitioner measures both these disk centers and then sits the patient up to make this observation will undoubtedly be an amazing experience and will confirm the exactness of the measurement methods given in this book. The posterior vortex disk is usually larger than the anterior vortex disk and probably reflects the more proximal location of the glands of origin of the chakras to the anterior body surface. As a result, the vortex is more spread as it crosses the posterior skin surface and the disk is of greater diamter. The color filters usually evoke a greater number of VAS pulse beats in the posterior chakra vortex than in the anterior vortex. The significance of this finding is not known.

The practitioner should keep the posterior vortex measurement in mind when providing balancing and, if convenient, the posterior vortexes should be balanced as well. The different vortex spin directions betwen male and female pertain to the posterior chakra vortexes as well as the anterior vortexes. In the male, the usual posterior chakra vortex spin is clockwise and in the female it is counter-clockwise.

When women of menstruation age are examined, it is important to know if they are menstruating. During menstruation, measurements are complicated by the fact that body energy oscillates in a random fashion and sometimes causes measurement values to vary. At the time of menstruation a well-defined vortex can be observed to arise over the region of the uterus.

Throughout this description discussion is directed toward the life energy field at chakra locations. It is believed that future investigations will identify disordered chakra patterns in most disease states. Once the practitioner has gained skill at chakra measurement and balancing, he is encouraged to begin measuring and balancing vortex fields around body regions of disorder. All body disorders seem to have an associated vortex.

All radiation and all physical matter has an energetic nature. Each quality of matter and each frequency of radiation possesses a discrete and characteristic level of resonance. The body energy field is equisitely sensitive to the resonance of radiation and matter. The physical body responds with observable reflexes instantaneously as radiation or matter enters its aura field. This response can be measured by the VAS. Radiation or matter to which the body can adapt (i.e., which can benefit the body) causes a positive VAS response. Matter to which the body cannot adapt (i.e., which is harmful) causes a negative VAS response.

It is a simple technique to place a substance such as an herb, food, drug or cell salt into the body field and to observe the VAS response. The heart chakra field is a convenient region in which to test substances, but any part of the aura can be used. One method is to bring the substance to the skin surface and to observe the VAS response. The substance need not be removed from its container if the container is glass and if the glass is free of toxic minerals such as lead.

An account of body energy balancing would be deficient if the healing effect of natural sunlight were omitted. Humans should obtain regular natural sunlight exposure for optimum energy balancing, as described in Kime, Zane R., M.D., **Sunlight Could Save Your Life**, World Health Publications, Penryn, California, 1980.

A simple two- to three-minute exercise called the cross-walk can be performed on a daily basis to balance the body subtle energy systems. A good practice might be to have all clients perform this exercise before each measurement session. The exercise is performed by jogging in place. Each time the left leg is lifted, the right arm should be in its forward upswing. Each time the right leg is lifted, the left arm should be in its forward upswing. In right-eye dominant individuals, the head should be turned 90 degrees to the right at each forward upswing of the right arm, and then turned straight forward art the forward upswing of the left arm. In left-eye dominant individuals, the head is turned 90 degrees to the left at the forward upswing of the left arm and straight forward at the forward upswing of the right argm. An individual can determine his eye dominance by punching a hole in a piece of paper with a pencil and then holding the paper at arm's length. Next an object or distant bright light is viewed through this hole, which is still at arm's length from the eyes. This visual focus is maintained on the object or light as the paper is slowly brought toward the nose. By the time the paper reaches the nose, the viewer will find that he is viewing the object with only one eye, the dominant eye.

### 6. Measuring the Ray.

The seventh chakra energy field emanates from the crown of the head. This field is called the ray and is thought to be related to the pineal gland. The pineal is known to be a light sensing organ filled with melanin, which is considered by some to be a transducer which steps down energy from the universal field into the individual body life field. Each individual's ray is a specific color or combination of colors. There are seven basic ray patterns. Jack Schwarz's book Human Energy Systems, E. P. Dutton, New York, 1980, provides excellent detail of the ray characteristics. Mr. Schwarz describes the character, personality and aptitude qualities of each ray. The ray is a sign of each person's emotional and mental composition and the ray remains constant throughout life.

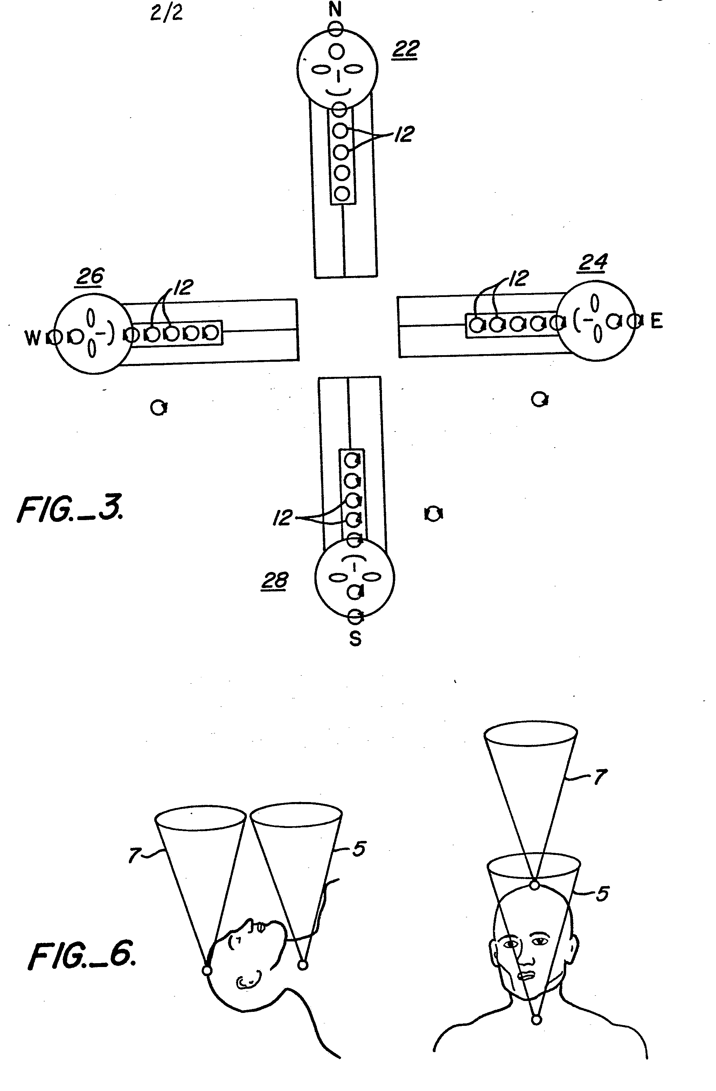

The spin of the ray can be determined by the methods described for the chakras. When making ray measurements on a client in a lying supine position, it is important to remember that all chakra vortexes (including the ray) originate in the body and radiate superiorly along an imaginary axis which originates in the center of the earth. Therefore the chakra 7 in a person who is lying down is actually directed as in Figure 6.

### 7. Crystal Healing.

Methods of crystal pulsing have been discovered and researched by Marcel Vogel. The quartz crystal functions as a uni-directional transducer of aura energy. The beam given by a hand-held pointed crystal has been described by Marcel Vogel in "Crystals, Healing and Medicine," from the Holmes Center Tenth Annual Symposium, **Science of Mind**, February, 1984, as a regularly structured beam of energy which can induce the restructuring of disorganized lattice and nidus patterns in the aura and body of a person who suffers disorder.

The preparation of the healing crystal is the same as has been described above. Once the beam has been generated between the hands of the practitioner it can be directed at the specific region of the client's energy disorder. The practitioner can then hold his left antenna hand on the opposite side of the client's body so as to feel the crystal beam as it passes through the client. The crystal is then turned slowly counter-clockwise until the antenna hand feels the beam "lock in." At this point the right hand holding the crystal can be rotated clockwise at one cycle per second in order to generate a crystal beam field in the region of the disorder. At each turn the crystal can be gently squeezed so as to pulse it. Natural quartz crystal is piezo-electric. During this pulsing activity the practitioner can take short deep inspirations, hold his breath, and then take long slow exhalations so as to enhance the energy intensity of his own aura field. The antenna hand will sense the regular pulsing of the crystal beam. While the pulsing is taking place, the client should be asked to participate in his own healing by mentally focusing on the cause of the disorder. The practitioner continues to pulse and build the healing field for another minute or two by continuing to rotate the right hand. Next the client is authoritatively commanded to take a deep breath and forcefully release his breath at the same time as the thought of causality is released. At this moment the crystal is abruptly drawn away and the practitioner also abruptly expells a forceful exhalation. At this point the client might experience a spontaneous emotional discharge which should not surprise the practitioner. For those problems which do not have a regional body fucus, the practitioner can utilize the heart chakra as a therapeutic witness area. The crystal healing technique can be used on any disordered chakra to rebalance it. Metaphysical studies will enhance the practitioner's understanding of the crystal healing techniques.

It should now be readily apparent to those skilled in the art that a novel method for treating an individual capable of achieving the stated objects of the invention has been provided. By using the VAS in a feedback loop to monitor the effect of stimuli introduced into an energy field vortex generated by the body, the stimuli can be applied in a controlled manner to balance the energy field vortexes of the body. Such balancing is a significant aid in prevention and amelioration of disease and related abnormal conditions.

It should further be apparent to those skilled in the art that various changes in form and details of the invention as shown and described may be made. It is intended that such changes be included within the spirit and scope of the claims appended hereto.

- 36 -

CLAIMS

1.   A combination for use in therapy to produce a positive change in the vasular autonomic signal of an individual, said combination comprising means for analysing an energy field vortex of an individual and means for positively stimulating an energy field vortex.

2.   A combination according to claim 1 in which the said means for positively stimulating an energy field vortex is means for providing light of a selected wavelength.

3.   A combination according to claim 1 in which the means for positively stimulating an energy field vortex is means for providing a magnetic field.

4.   A combination according to claim 1 in which the said means for positively stimulating an energy field vortex is means for providing an electrical signal.

5.   A combination according to claim 1 in which the said means for positively stimulating an energy field vortex is means for providing sound.

6.   A combination according to claim 1 in which the said means for positively stimulating an energy field vortex is a crystal configured to transduce a human aura.

7.   A combination according to claim 1 in which the said means for positively stimulating an energy field vortex is a flower essence.

8.   A combination according to claim 1 in which the said means for positively stimulating an energy field vortex is a cell salt.

9.   A combination according to claim 1 in which the said means for positively stimulating an energy field vortex is a plurality of metal pellets.

10.   A combination according to any of claims 1 to 9 in which the said means for analysing an energy field vortex of an individual comprises a quartz crystal or a magnet.

0164836

1/2

FIG._1.

FIG._2.

FIG._4.

FIG._5.

0164836

N

22

12

26

12

24

12

W

E

O

O

FIG._3.

12

28

S

7

7

5

5

FIG._6.

**European Patent Office**

**EUROPEAN SEARCH REPORT**

## DOCUMENTS CONSIDERED TO BE RELEVANT

EP 85302403.2

| Category | Citation of document with indication, where appropriate, of relevant passages | Relevant to claim | CLASSIFICATION OF THE APPLICATION (Int. Cl 4) |
|---|---|---|---|
| A | DE - A1 - 2 947 194 (MERSMANN) <br> * Page 1, claim 1; page 4, claim 7 * | 1,10 | A 61 N 1/00 <br><br> A 61 N 5/00 <br><br> A 61 H 39/00 |
| A | DE - A1 - 2 442 487 (PRINZ) <br> * Totality * | 1-10 | |
| A | EP - A2 - 0 058 105 (JAVEILE) <br> * Abstract; page 6, line 37 - page 7, line 31 * | 1,2 | |
| A | FR - A1 - 2 514 257 (CESKOSLO- VENSKA ADADEMIE) <br> * Page 3, lines 22-35; fig. 1-3 * | 1,2 | |
| A | FR - A1 - 2 329 258 (MESSERSCHMITT-BOLKOW-BLOHM) <br> * Page 4, lines 7-20; fig. 1,2 * | 1,2 | **TECHNICAL FIELDS SEARCHED (Int. Cl.4)** <br><br> A 61 N <br><br> A 61 H |
| A | GB - A - 1 600 217 (SKOVAJSA) <br> * Page 1, lines 8-31 * | 1,2 | |
| A | DD - A - 155 876 (VEB INGENIEURBUERO) <br> * Abstract; page 4, claim * | 1,2 | |

The present search report has been drawn up for all claims

| Place of search | Date of completion of the search | Examiner |
|---|---|---|
| VIENNA | 13-06-1985 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

| | DOCUMENTS CONSIDERED TO BE RELEVANT | | EP 85302403.2 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | EP - A2 - 0 100 050 (BLUM)<br>* Abstract; page 1, line 6 - page 2, line 17 * | 1,3 | |
| A | DE - A1 - 3 220 718 (GEISSER)<br>* Page 2, lines 1-13 * | 1,3 | |
| A | DE - A1 - 2 928 826 (PAPE)<br>* Page 1, claims 1-3 * | 1,10 | |
| A | DE - B2 - 2 659 115 (LUDWIG)<br>* Column 1; claim 1 * | 1,3,10 | |
| A | DE - A - 2 235 015 (PRINZ)<br>* Page 4, claims 1-3 * | 1,3 | TECHNICAL FIELDS SEARCHED (Int. Cl.4) |
| A | AT - B - 352 866 (LOHER)<br>* Page 3, claims 1,2; fig. * | 1,4 | |
| A | DE - A1 - 3 218 843 (KNAPP)<br>* Page 1, lines 1-19 * | 1,4 | |
| A | CH - A5 - 620 821 (STANLEY ELECTRIC)<br>* Abstract * | 1,4 | |

The present search report has been drawn up for all claims

| Place of search | Date of compilation of the search | Examiner |
|---|---|---|
| VIENNA | 13-06-1985 | NEGWER |

**European Patent Office**

**EUROPEAN SEARCH REPORT**

| | DOCUMENTS CONSIDERED TO BE RELEVANT | | EP 85302403.2 |
|---|---|---|---|

| Category | Citation of document with indication, where appropriate, of relevant passages | Relevant to claim | CLASSIFICATION OF THE APPLICATION (Int. Cl 4) |
|---|---|---|---|
| A | FR − A1 − 2 500 745 (DERVIEUX) <br> * Page 2, lines 16-38 * <br> -- | 1,4,6 | |
| A | FR − A1 − 2 285 019 (BIOENGENEE-RING) <br> * Page 1, lines 8-17; fig. 1 * <br> -- | 1,5 | |
| A | DE − A1 − 2 551 159 (SEUSS) <br> * Page 5, claim 21; page 16, paragraph * <br> ---- | 1,7 | |
| | | | TECHNICAL FIELDS SEARCHED (Int. Cl.4) |

The present search report has been drawn up for all claims

| Place of search <br> VIENNA | Date of completion of the search <br> 13-06-1985 | Examiner <br> NEGWER |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82